# EUROPEAN PATENT SPECIFICATION

(11) **EP 2 906 776 B1**
(45) Date of publication and mention of the grant of the patent: **08.05.2019**
(21) Application number: 13847702.1
(22) Date of filing: 15.10.2013
(51) Int. Cl.: E21B 21/08, E21B 21/01, G01N 11/04, G01N 33/28

(54) **METHOD AND APPARATUS FOR TESTING LOST CIRCULATION MATERIALS WITHIN A PERMEABILITY PLUGGING APPARATUS**
VERFAHREN UND VORRICHTUNG ZUM TESTEN VON LOST-CIRCULATION-STOFFEN IN EINER PERMEABILITÄTSABDICHTUNGSVORRICHTUNG
MÉTHODE ET APPAREIL DE TEST DE MATÉRIAUX COLMATANTS DANS UN APPAREIL DE COLMATAGE DE PERMÉABILITÉ

(30) Priority: 15.10.2012 US 201213652142
(43) Date of publication of application: 19.08.2015
(73) Proprietor: Halliburton Energy Services, Inc., Houston, TX 77072 (US)
(72) Inventor: MURPHY, Robert, J., Kingwood, TX 77339 (US); MILLER, Matthew, L., Spring, TX 77381 (US)
(74) Representative: Turner, Craig Robert
(86) International application number: PCT/US2013/064961
(87) International publication number: WO 2014/062626

(56) References cited:
- EP-A1- 2 810 062
- WO-A1-2008/118953
- US-A- 4 748 849
- US-A1- 2009 029 878
- US-A1- 2009 029 878
- US-A1- 2011 290 012
- US-B2- 7 870 782
- US-B2- 8 091 726
- HAMIDA T ET AL: "Filtration loss characteristics of aqueous waxy hull-less barley (WHB) solutions", JOURNAL OF PETROLEUM SCIENCE AND ENGINEERING, ELSEVIER, AMSTERDAM, NL, vol. 72, no. 1-2, 1 May 2010 (2010-05-01), pages 33-41, XP027046911, ISSN: 0920-4105 [retrieved on 2010-02-24]
- "PERMEABIILIITY PLUGGIING APPARATUS (PPA)", , 1 January 2007 (2007-01-01), XP055060626, Houston, Texas, USA Retrieved from the Internet: URL:http://www.fann.com/public1/products/p ubsdata/Brochures/PPA.pdf [retrieved on 2013-04-22]

## Description

### BACKGROUND

The present disclosure relates generally to well drilling operations and, more particularly, to a method and apparatus for testing lost circulation materials (LCMs) in a permeability plugging apparatus (PPA).

Subterranean drilling operations typically utilize drilling fluids to provide hydrostatic pressure to prevent formation fluids from entering into the well bore, to keep the drill bit cool and clean during drilling, to carry out drill cuttings, and to suspend the drill cuttings while drilling is paused and when the drilling assembly is brought in and out of the borehole. When drilling into certain formation types, some of the drilling fluid may seep into and become trapped in the formation. This is particularly problematic in vugular formations, which include numerous cavities, known as vugs. If enough drilling fluid is lost to the formation, additional drilling fluid must be introduced into the borehole to maintain drilling efficiency. This can become expensive if large amounts of the drilling fluid are lost.

To prevent drilling fluid loss into vugular formations, LCMs may be added to the drilling fluid. The LCMs typically are typically fibrous (e.g., cedar bark, shredded cane stalks, mineral fiber and hair), flaky (e.g., mica flakes and pieces of plastic or cellophane sheeting) or granular (e.g., ground and sized limestone or marble, wood, nut hulls, Formica, corncobs and cotton hulls) materials. In certain other instances, LCMs may include reactive chemicals which set and harden within the vugs. The LCMs are intended to plug the vugs, preventing the vugs from capturing the fluid portions of the drilling fluid. Unfortunately, testing the effectiveness of LCMs can be problematic. For example, current testing apparatuses, which include permeability plugging apparatuses (PPAs) and high-temperature, high-pressure systems (HTHPs), typically cannot simulate large vugs due to apparatus limitations that cause them to become clogged before the effectiveness of the LCM material can be determined. Additionally, modifications to the testing apparatuses can be dangerous given the high temperatures and pressures that are required as part of the testing procedures.

Filtration loss characteristics of aqueous waxy hull-less barley (WHB) solutions; Journal of Petroleum Science and Engineering - May 2010, Vol 72, Nr:1-2, Pages 33-41 discloses a typical PPA test cell arrangement.

### FIGURES

Some specific exemplary embodiments of the disclosure may be understood by referring, in part, to the following description and the accompanying drawings.
Figure 1 illustrates an example PPA cell.
Figure 2 illustrates an example apparatus according to aspects of the present disclosure.
Figure 3 illustrates a cross-section of an example apparatus according to aspects of the present disclosure.

### DETAILED DESCRIPTION

The present disclosure relates generally to well drilling operations and, more particularly, to a method and apparatus for testing LCMs in a PPA.

Illustrative embodiments of the present disclosure are described in detail herein. In the interest of clarity, not all features of an actual implementation may be described in this specification. It will of course be appreciated that in the development of any such actual embodiment, numerous implementation-specific decisions must be made to achieve the specific implementation goals, which will vary from one implementation to another. Moreover, it will be appreciated that such a development effort might be complex and time-consuming, but would nevertheless be a routine undertaking for those of ordinary skill in the art having the benefit of the present disclosure.

To facilitate a better understanding of the present disclosure, the following examples of certain embodiments are given. In no way should the following examples be read to limit, or define, the scope of the disclosure. Embodiments of the present disclosure may be useful in hydrocarbon drilling operations in a variety of different settings. Embodiments described below with respect to one implementation are not intended to be limiting.

According to aspects of the present disclosure, an apparatus for testing LCMs within a PPA is described. The apparatus may include a PPA cell cap and an elongated LCM receiver. At least one tubular member may provide fluid communication between the PPA cell cap and the elongated LCM receiver. The at least one tubular member may be sized to accommodate large particulate LCM testing within the PPA. As will be discussed below, large particulate LCM testing within the PPA may include testing particulates that are designed or intended to plug vugs or slots within a PPA cell with a width of greater than or equal to one millimeter. In certain embodiments, the at least one tubular member may have a bore larger than the bore of typical PPA equipment, so as to accommodate the large particulate LCMs without becoming clogged. Likewise, the apparatus may also include a backpressure inlet through the elongated LCM receiver. As will also be discussed below, providing back pressure within the LCM receiver may extend the temperature and pressure ranges under which the large particulate LCMs may be tested.

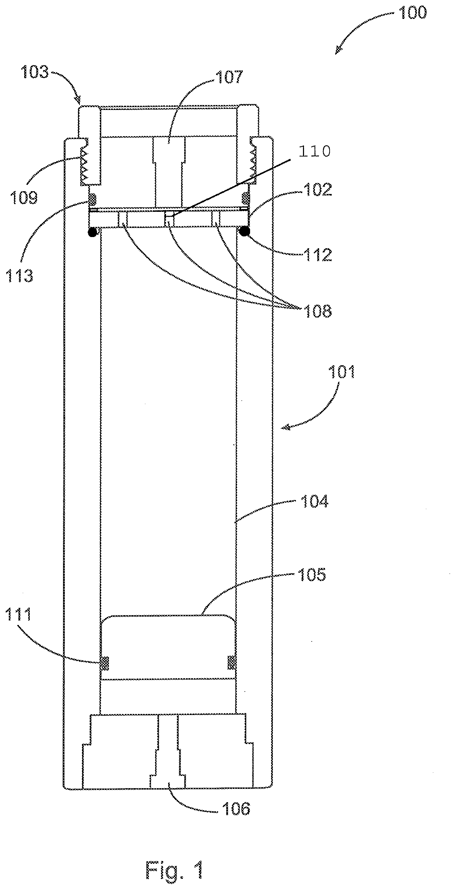

Fig. 1 shows an example PPA cell 100. The PPA cell 100 may include a PPA cell body 101 and a PPA cell cap 103 coupled to the PPA cell body 101 via a threaded engagement 109. A slotted disc 102 may be placed within the PPA cell body 101 and held in place by the PPA cell cap 103. The slotted disc 102 may be in fluid communication with the exterior of the PPA cell 100 through a hole 107 in the PPA cell cap 103. In operation, fluids containing LCM materials may be placed in chamber 104. A floating piston 105 may be disposed within the chamber 104, and act as a barrier between the LCM fluids and the port 106 in the bottom of the PPA cell body 101. O-ring 111 may be disposed within a groove on an outer portion of the piston 105, sealing the portion of the chamber 104 above the piston 105 from the portion of the chamber 104 below the piston 105. A pressurization mechanism may be connected with the PPA cell 100 via the port 106 and may pressurize the portion of the chamber 104 opposite the LCM fluids. This pressurization may force the piston 105 upwards, causing the LCM fluids to travel through slots 108 in the slotted disc 102. In certain embodiments, as can be seen, O-rings 112 and 113 may ensure that the LCM fluid flows through the slotted disc 102 and the hole 107.

In typical LCM testing scenarios, the width of the slots 110 may be less than one millimeter. In such instances, the LCM fluids may travel though the slots and out the hole 107 until the slots 110 become "plugged", thereby allowing the effectiveness of the LCM fluids to be determined. When the width of the slots 108 is increased-for example, to one millimeter or greater-the size of the particulates in the LCM fluid must also be increased. In existing PPA equipment, however, the large particulates those intended or designed to plug a vug or slot with a width greater than or equal to one millimeter in the LCM fluid typically causes the hole 107 to clog before the slots 108 can become "plugged", preventing the effectiveness of the large particulate LCM fluid from being determined. Additionally, modifications to the existing PPA equipment are difficult, as changes to the size of the hole or the bore of corresponding tubes can decrease the temperature or pressure range under which the large particulate LCM fluid may be tested.

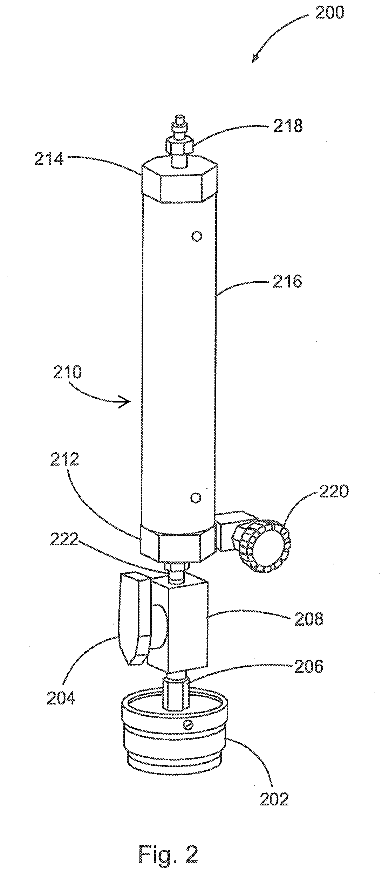

Fig. 2 shows an example apparatus 200 for testing large particulate LCMs within a PPA, according to aspects of the present disclosure. The apparatus 2 comprises a PPA cell cap 202 and an elongated LCM receiver 210. The PPA cell cap 202 may be a typical PPA cell cap modified to accommodate a larger bore opening than in a typical PPA cell cap. At least one tubular member 204 provides fluid communication between the PPA cell cap 202 and the elongated LCM receiver 210. The at least one tubular member 204 is sized to accommodate large particulate LCM testing within the PPA. This includes having a bore larger than typical PPA equipment, and more specifically with a bore large enough to accommodate large particulate LCM materials without clogging, including the LCM materials intended or designed to plug slots or vugs greater than or equal to one millimeter in width. Additionally, the apparatus 200 includes a backpressure inlet 218 through the elongated LCM receiver 210.

The at least one tubular member 204 may be comprised of multiple tubular members that define a substantially constant internal bore. In the embodiment shown, the at least one tubular member 204 includes nipples 206 and 222 and valve 208. The at least one tubular member 204 may be coupled via threaded connections to both the PPA cell cap 202 and the elongated LCM receiver 210. In the embodiment shown, the nipple 206 may be coupled to the PPA cell cap 202 via a threaded connection and nipple 222 may be coupled to the elongated receiver 210 via a threaded engagement. Similarly, the valve 208 may be positioned between and coupled to both nipple 206 and nipple 222 via threaded engagement. Other coupling types are possible, however, as would be appreciated by one of ordinary skill in view of this disclosure.

The elongated LCM receiver 210 may comprise a first end cap 214, a second end cap 212, and a cylindrical receiver body 216. The first end cap 214 and the second end cap 212 may be coupled to the cylindrical receiver body 216 via a threaded connection. In certain embodiments, the backpressure inlet 218 may be disposed within the first end cap 214 and the at least one tubular member 204 may be coupled to the second end cap 212 and may provide fluid communication between the PPA cell cap 202 and the elongated LCM receiver through the second end cap 212. As described above, a backpressure may be introduced into the elongated LCM receiver 210 via the backpressure inlet 218. When large bore tubular members, such as the at least one tubular member 204, are introduced between the PPA cell cap 202 and the elongated LCM receiver 210, introducing a back pressure may increase the temperature range under which the LCM material may be tested.

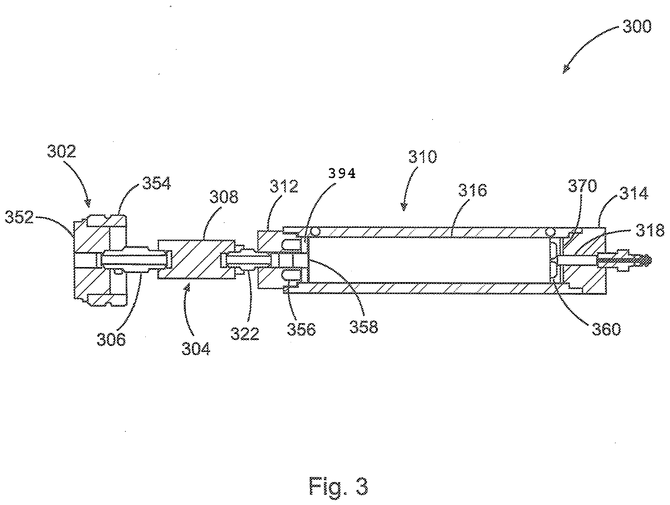

In certain embodiments a test valve 220 may be coupled to the elongated LCM receiver 210. LCM fluids that have passed through the PPA cell cap 202 and the at least one tubular member 204 may be received within the elongated LCM receiver 210. The test valve 220 may be used to extract the LCM fluids within the elongated LCM receiver 210 for testing purposes. For example, the volume of the fluid that passes through the slotted disc 102 may measured with respect to time by periodically emptying the fluid from the receiver and measuring its volume. In certain embodiments, as will be discussed below with respect to Fig. 3, the elongated LCM receiver 210 may include internal structures, such as screens and deflectors, to reduce the likelihood that the test valve 220 will become clogged by the particulates in the LCM fluid.

Fig. 3 illustrates a cross-section of an example apparatus according to aspects of the present disclosure. The apparatus 300 comprises a PPA cell cap 302 and an elongated LCM receiver 310. At least one tubular member 304 provides fluid communication between the PPA cell cap 302 and the elongated LCM receiver 310. The at least one tubular member 304 is sized to accommodate large particulate LCM testing within the PPA. This includes having a bore large enough to accommodate large particulate LCM. Additionally, the apparatus 300 may include a backpressure inlet 318 through the elongated LCM receiver 310.

The PPA cell cap 302 may comprise a cap 352 sized to engage with a PPA cell and a threaded retainer ring 354 at least partially disposed around the cap 352. The threaded retainer ring 354 may be sized to engage with the internal threads in the PPA cell. The at least one tubular member 304 may comprise nipples 306 and 322, and valve 308. Nipple 306 may be coupled to the PPA cell cap 302 and nipple 322 may be coupled to the elongated LCM receiver 310. The valve 308 may be positioned between and coupled to both the nipple 306 and 322.

The elongated LCM receiver may comprise a first end cap 314 and second end cap 312. The end caps 312 and 314 may be coupled and secured to a receiver body 316 via threaded engagement or other coupling techniques known in the art. The nipple 322 may be coupled to the second end cap 312, providing fluid communication between the PPA cell cap 302 and an internal chamber within the receiver body 316. A test valve (not shown) may be coupled to the elongated LCM receiver 310 and provide fluid communication with the elongated LCM receiver 310 through a fluid communication pathway. The fluid communication pathway may be at least partially disposed within the second end cap 312, and include chamber 356. For example, fluid may pass from chamber 356 through a port (not shown) to the test valve. In certain embodiments, a screen 394 may be positioned proximate to the fluid pathway. The screen may be secured in place via a retainer 358 such that the screen 394 does not move axially within the elongated LCM receiver 310.

In certain embodiments, LCM fluid may be received within the receiver body 316 through the second end cap 312. Fluid samples may be taken from the elongated LCM receiver 310 via a test valve. The LCM fluid within the receiver body 316 may pass through the screen 394 before settling in the chamber 356, where they can exit through a fluid passageway with the test valve. The screen 394 may capture particulates from the LCM fluid, preventing the particulates from settling within in the chamber 356. This may reduce the likelihood that the fluid passageway and test valve would become clogged from the particulate matter within the LCM fluid.

In certain embodiments, a backpressure inlet 318 may be disposed within the first end cap 314. As the LCM fluids are received within the elongated LCM receiver 310, pressure from the PPA cell may force the fluid upwards towards the backpressure inlet 318. The rapid initial flow of the LCM fluid into the receiver may jet up to the top, clogging the backpressure inlet 318. In the present embodiment, a deflector 360 is positioned within the elongated LCM receiver 310 proximate to the first end cap 314. In certain embodiment, the deflector may be integrated with the first end cap 314. As the LCM fluids are received within the elongated LCM receiver 310, the deflector 360 may reduce the likelihood that the backpressure inlet becomes clogged. A passage 370, in first end cap 314, may connect to the backpressure inlet 318 and allow control of the pressure in the chamber 316. The passage 370 may connects to a small annular clearance between the deflector 360 and the interior wall of the chamber 316.

According to the invention, a method for testing LCMs within a PPA is also described herein. The method comprises the step of positioning a slotted disc within a PPA cell. The PPA cell and slotted disc may be similar to the PPA cell and slotted disc shown in Fig. 1. According to the invention, the slotted disc may comprise at least one slot with a width greater than or equal to one millimeter. The PPA cell is used to test large particulate LCMs, including LCMs designed or intended to plug the at least one slot with a width greater than or equal to one millimeter.

The method also includes the step of coupling an LCM receiver apparatus to the PPA cell. Coupling may comprise direct or indirect connection and may include a threaded connection. In certain embodiments, the LCM receiver apparatus may comprise an apparatus similar to the apparatus described with respect to Figs. 2 and 3. The LCM receiver apparatus comprises a PPA cell cap, an elongated LCM receiver, at least one tubular member providing fluid communication between the PPA cell and the elongated LCM receiver, and a backpressure inlet through the elongated LCM receiver. The at least one tubular member is sized to accommodate large particulate LCM testing within the PPA.

In certain embodiments, the elongated LCM receiver may comprise a first end cap and a second end cap. The backpressure inlet may be disposed in the first end cap. The method may also include the step of applying a backpressure to the LCM receiver assembly, which may comprise applying the pressure through the backpressure inlet. Additionally, the at least one tubular member may provide fluid communication between the PPA cell cap and the second end cap. As described above, applying a back pressure within the elongated LCM receiver apparatus may increase the temperature testing range of the PPA when a tubular member with a large bore is used.

In certain embodiments, the LCM receiver apparatus may also include a test valve coupled to and providing fluid communication with the elongated LCM receiver through a fluid communication pathway. The LCM receiver apparatus may also comprise a screen positioned within the elongated LCM receiver proximate to the fluid communication pathway. As described above, periodically removing LCM fluids from the elongated LCM receiver may be necessary for testing purposes. By positioning the screen proximate to the fluid passageway, particulates within the LCM fluid may be prevented from clogging the test valve.

Therefore, the present disclosure is well adapted to attain the ends and advantages mentioned as well as those that are inherent therein. The particular embodiments disclosed above are illustrative only, as the present disclosure may be modified and practiced in different but equivalent manners apparent to those skilled in the art having the benefit of the teachings herein. Furthermore, no limitations are intended to the details of construction or design herein shown, other than as described in the claims below. Also, the terms in the claims have their plain, ordinary meaning unless otherwise explicitly and clearly defined by the patentee. The indefinite articles "a" or "an," as used in the claims, are defined herein to mean one or more than one of the element that it introduces.

## Claims

1. A lost circulation materials (LCM) receiver apparatus (200, 300) for use in testing LCM within a permeability plugging apparatus (PPA) cell (101), the LCM receiver apparatus comprising:
a PPA cell cap (103, 202, 302);
an elongated LCM receiver (210, 310);
at least one tubular member (204, 304) providing fluid communication between the PPA cell cap (103, 202, 302) and the elongated LCM receiver (210, 310), wherein the at least one tubular member (204, 304) comprises a bore large enough to accommodate large particulate LCM testing within the PPA (100), without becoming clogged, wherein the large particulate LCM are intended or designed to plug slots (108) within said permeability plugging apparatus (PPA) cell (101) having - a width of greater than or equal to one millimeter; and
a backpressure inlet (218, 318) through the elongated LCM receiver (210, 310).

2. The apparatus of claim 1, wherein the elongated LCM receiver (210, 310) comprises a first end cap (214, 314) and a second end cap (212, 312).

3. The apparatus of claim 2, wherein the backpressure inlet (218, 318) is disposed in the first end cap (214, 314), optionally wherein the at least one tubular member (204, 304) provides fluid communication between the PPA cell cap 103, 202, 302) and the second end cap (212, 312).

4. The apparatus of claim 1, further comprising a test valve (220) coupled to and providing fluid communication with the elongated LCM receiver (210, 310) through a fluid communication pathway, optionally further comprising a screen (394) positioned within the elongated LCM receiver (210, 310) so as to prevent clogging of the test valve (220).

5. The apparatus of claim 1, further comprising a deflector (360) positioned within the elongated LCM receiver (210, 310) so as to prevent clogging of the backpressure inlet (218, 318).

6. A method for testing lost circulation materials (LCM) within a permeability plugging apparatus (PPA) cell (101), comprising:
positioning a slotted disk (102) within a PPA cell (101), wherein the slotted disk (102) comprises at least one slot (108) with a width greater than or equal to one millimeter;
coupling an LCM receiver apparatus (200, 300) to the PPA cell (101), where in the LCM receiver apparatus (200, 300) further comprises:
a PPA cell cap (103, 202, 302);
an elongated LCM receiver (210, 310);
at least one tubular member (204, 304) providing fluid communication between the PPA cell (101) and the elongated LCM receiver (210, 310), wherein the at least one tubular member (204, 304) comprises a bore large enough to accommodate large particulate LCM testing within the PPA (100), without becoming clogged; wherein the large particulate LCM are intended or designed to plug slots (108) within said permeability plugging apparatus (PPA) cell (101) having a width of greater than or equal to one millimeter, and
a backpressure inlet (218, 318) through the elongated LCM receiver (210, 310);
applying a backpressure to the LCM receiver apparatus (200, 300).

7. The method of claim 6, wherein the elongated LCM receiver (210, 310) comprises a first end cap (214, 314) and a second end cap (212, 312).

8. The method of claim 7, further comprising any of the following:
(i) wherein the backpressure inlet (218, 318) is disposed in the first end cap (214, 314); and wherein applying the backpressure to the LCM receiver apparatus (200, 300) comprises applying the pressure through the backpressure inlet (218, 318);
(ii) wherein the at least one tubular member (204, 304) provides fluid communication between the PPA cell cap (103, 202, 302); and the second end cap (212, 312).

9. The method of claim 6, wherein the LCM receiver apparatus (200, 300) further comprises:
a test valve (220) coupled to and providing fluid communication with the elongated LCM receiver (210, 310) through a fluid communication pathway; and
a screen (394) positioned within the elongated LCM receiver (210, 310) so as to prevent clogging of the test valve.

## Patentansprüche

1. Aufnahmevorrichtung (200, 300) für Lost-Circulation-Stoffe (lost circulation materials - LCM) zur Verwendung beim Testen von LCM in einer Zelle (101) einer Permeabilitätsabdichtungsvorrichtung (permeability plugging apparatus - PPA), wobei die LCM-Aufnahmevorrichtung Folgendes umfasst:
einen PPA-Zellenverschluss (103, 202, 302);
einen langgestreckten LCM-Aufnehmer (210, 310);
zumindest ein rohrförmiges Element (204, 304), das eine Fluidkommunikation zwischen dem PPA-Zellenverschluss (103, 202, 302) und dem langgestreckten LCM-Empfänger (210, 310) bereitstellt, wobei das zumindest eine rohrförmige Element (204, 304) eine Bohrung umfasst, die groß genug ist, um ein Testen von LCM mit großen Teilchen in der PPA (100) zu ermöglichen, ohne zu verstopfen, wobei die LCM-Stoffe mit großen Teilchen vorgesehen oder entworfen sind, um Schlitze (108) in der Zelle (101) der Permeabilitätsabdichtungsvorrichtung (permeability plugging apparatus - PPA) abzudichten, die eine Breite von mehr als oder gleich einem Millimeter aufweisen; und
einen Gegendruckeinlass (218, 318) durch den langgestreckten LCM-Aufnehmer (210, 310).

2. Vorrichtung nach Anspruch 1, wobei der langgestreckte LCM-Aufnehmer (210, 310) einen ersten Endverschluss (214, 314) und einen zweiten Endverschluss (212, 312) umfasst.

3. Vorrichtung nach Anspruch 2, wobei der Gegendruckeinlass (218, 318) in dem ersten Endverschluss (214, 314) angeordnet ist, wobei das zumindest eine rohrförmige Element (204, 304) gegebenenfalls eine Fluidkommunikation zwischen dem PPA-Zellenverschluss 103, 202, 302) und dem zweiten Endverschluss (212, 312) bereitstellt.

4. Vorrichtung nach Anspruch 1, ferner umfassend ein Testventil (220), das durch einen Fluidkommunikationsdurchgang an den langgestreckten LCM-Aufnehmer (210, 310) gekoppelt ist und eine Fluidkommunikation mit diesem bereitstellt, gegebenenfalls ferner umfassend ein Sieb (394), das in dem langgestreckten LCM-Aufnehmer (210, 310) positioniert ist, um ein Verstopfen des Testventils (220) zu verhindern.

5. Vorrichtung nach Anspruch 1, ferner umfassend eine Ablenkeinrichtung (360), die in dem langgestreckten LCM-Aufnehmer (210, 310) positioniert ist, um ein Verstopfen des Gegendruckventils (218, 318) zu verhindern.

6. Verfahren zum Testen von Lost-Circulation-Stoffen (lost circulation materials - LCM) in einer Zelle (101) einer Permeabilitätsabdichtungsvorrichtung (permeability plugging apparatus - PPA), umfassend:
Positionieren einer geschlitzten Scheibe (102) in einer PPA-Zelle (101), wobei die geschlitzte Scheibe (102) zumindest einen Schlitz (108) mit einer Breite von größer als oder gleich einem Millimeter umfasst;
Koppeln einer LCM-Aufnahmevorrichtung (200, 300) an die PPA-Zelle (101), wobei die LCM-Aufnahmevorrichtung (200, 300) ferner Folgendes umfasst:
einen PPA-Zellenverschluss (103, 202, 302);
einen langgestreckten LCM-Aufnehmer (210, 310);
zumindest ein rohrförmiges Element (204, 304), das eine Fluidkommunikation zwischen der PPA-Zelle (101) und dem langgestreckten LCM-Empfänger (210, 310) bereitstellt, wobei das zumindest eine rohrförmige Element (204, 304) eine Bohrung umfasst, die groß genug ist, um ein Testen von LCM mit großen Teilchen in der PPA (100) zu ermöglichen, ohne zu verstopfen, wobei die LCM-Stoffe mit großen Teilchen vorgesehen oder entworfen sind, um Schlitze (108) in der Zelle (101) der Permeabilitätsabdichtungsvorrichtung (permeability plugging apparatus - PPA) abzudichten, die eine Breite von mehr als oder gleich einem Millimeter aufweisen; und
einen Gegendruckeinlass (218, 318) durch den langgestreckten LCM-Aufnehmer (210, 310);
Ausüben eines Gegendrucks auf die LCM-Aufnahmevorrichtung (200, 300) .

7. Verfahren nach Anspruch 6, wobei der langgestreckte LCM-Aufnehmer (210, 310) einen ersten Endverschluss (214, 314) und einen zweiten Endverschluss (212, 312) umfasst.

8. Verfahren nach Anspruch 7, ferner umfassend eines der Folgenden:
(i) wobei der Gegendruckeinlass (218, 318) in dem ersten Endverschluss (214, 314) angeordnet ist; und wobei das Ausüben des Gegendrucks auf die LCM-Aufnahmevorrichtung (200, 300) Ausüben des Drucks durch den Gegendruckeinlass (218, 318) umfasst;
(ii) wobei das zumindest eine rohrförmige Element (204, 304) eine Fluidkommunikation zwischen dem PPA-Zellenverschluss (103, 202, 302) und dem zweiten Endverschluss (212, 312) bereitstellt.

9. Verfahren nach Anspruch 6, wobei die LCM-Aufnahmevorrichtung (200, 300) ferner Folgendes umfasst:
ein Testventil (220), das durch einen Fluidkommunikationsdurchgang an den langgestreckten LCM-Aufnehmer (210, 310) gekoppelt ist und eine Fluidkommunikation mit diesem bereitstellt; und
ein Sieb (394), das in dem langgestreckten LCM-Aufnehmer (210, 310) positioniert ist, um ein Verstopfen des Testventils zu verhindern.

## Revendications

1. Appareil récepteur (200, 300) de matériaux colmatants (LCM) destiné à être utilisé pour le test de LCM à l'intérieur d'une cellule (101) d'appareil de colmatage de perméabilité (PPA), l'appareil récepteur de LCM comprenant :
un capuchon de cellule de PPA (103, 202, 302) ;
un récepteur de LCM allongé (210, 310) ;
au moins un élément tubulaire (204, 304) fournissant une communication fluidique entre le capuchon de cellule de PPA (103, 202, 302) et le récepteur de LCM allongé (210, 310), dans lequel l'au moins un élément tubulaire (204, 304) comprend un alésage suffisamment grand pour loger un test de LCM à grandes particules à l'intérieur du PPA (100) sans devenir bouché, dans lequel les matériaux LCM à grandes particules sont destinés ou conçus pour colmater des fentes (108) à l'intérieur de ladite cellule (101) d'appareil de colmatage de perméabilité (PPA) présentant une largeur supérieure ou égale à un millimètre ; et
une entrée de contre-pression (218, 318) à travers le récepteur de LCM allongé (210, 310).

2. Appareil selon la revendication 1, dans lequel le récepteur de LCM allongé (210, 310) comprend un premier capuchon d'extrémité (214, 314) et un second capuchon d'extrémité (212, 312) .

3. Appareil selon la revendication 2, dans lequel l'entrée de contre-pression (218, 318) est disposée dans le premier capuchon d'extrémité (214, 314), éventuellement dans lequel l'au moins un élément tubulaire (204, 304) fournit une communication fluidique entre le capuchon de cellule de PPA (103, 202, 302) et le second capuchon d'extrémité (212, 312).

4. Appareil selon la revendication 1, comprenant en outre une vanne de test (220) couplée à et fournissant une communication fluidique avec le récepteur de LCM allongé (210, 310) à travers un chemin de communication fluidique, comprenant éventuellement en outre un écran (394) positionné à l'intérieur du récepteur de LCM allongé (210, 310) de manière à empêcher le bouchage de la vanne de test (220).

5. Appareil selon la revendication 1, comprenant en outre un déflecteur (360) positionné à l'intérieur du récepteur de LCM allongé (210, 310) de manière à empêcher le bouchage de l'entrée de contre-pression (218, 318).

6. Procédé de test de matériaux colmatants (LCM) à l'intérieur d'une cellule (101) d'appareil de colmatage de perméabilité (PPA), comprenant :
le positionnement d'un disque à fentes (102) à l'intérieur d'une cellule (101) de PPA, dans lequel le disque à fentes (102) comprend au moins une fente (108) avec une largeur supérieure ou égale à un millimètre ;
le couplage d'un appareil récepteur de LCM (200, 300) à la cellule (101) de PPA, dans lequel l'appareil récepteur de LCM (200, 300) comprend en outre :
un capuchon de cellule de PPA (103, 202, 302) ;
un récepteur de LCM allongé (210, 310) ;
au moins un élément tubulaire (204, 304) fournissant une communication fluidique entre la cellule de PPA (101) et le récepteur de LCM allongé (210, 310), dans lequel l'au moins un élément tubulaire (204, 304) comprend un alésage suffisamment grand pour loger un test de LCM à grandes particules à l'intérieur du PPA (100) sans devenir bouché ; dans lequel le LCM à grandes particules est destiné ou conçu pour colmater des fentes (108) à l'intérieur de ladite cellule (101) d'appareil de colmatage de perméabilité (PPA) présentant une largeur supérieure ou égale à un millimètre, et
une entrée de contre-pression (218, 318) à travers le récepteur de LCM allongé (210, 310) ;
l'application d'une contre-pression à l'appareil récepteur de LCM (200, 300).

7. Procédé selon la revendication 6, dans lequel le récepteur de LCM allongé (210, 310) comprend un premier capuchon d'extrémité (214, 314) et un second capuchon d'extrémité (212, 312) .

8. Procédé selon la revendication 7, comprenant l'un quelconque des éléments suivants :
(i) dans lequel l'entrée de contre-pression (218, 318) est disposée dans le premier capuchon d'extrémité (214, 314) ; et dans lequel l'application de la contre-pression à l'appareil récepteur de LCM (200, 300) comprend l'application de la pression à travers l'entrée de contre-pression (218, 318) ;
(ii) dans lequel l'au moins un élément tubulaire (204, 304) fournit une communication fluidique entre le capuchon de cellule de PPA (103, 202, 302) ; et le second capuchon d'extrémité (212, 312) .

9. Procédé selon la revendication 6, dans lequel l'appareil récepteur de LCM (200, 300) comprend en outre :
une vanne de test (220) couplée à et fournissant une communication fluidique avec le récepteur de LCM allongé (210, 310) à travers un chemin de communication fluidique ; et
un écran (394) positionné à l'intérieur du récepteur de LCM allongé (210, 310) de manière à empêcher le bouchage de la vanne de test.
